# EUROPÄISCHE PATENTSCHRIFT

(11) **EP 3 346 954 B1**
(45) Veröffentlichungstag und Bekanntmachung des Hinweises auf die Patenterteilung: **22.11.2023**
(21) Anmeldenummer: 16775480.3
(22) Anmeldetag: 09.09.2016
(51) Int. Cl.: A61F 2/915

(54) **EXPANDIERBARE GEFÄSSSTÜTZE**
EXPANDABLE VASCULAR STENT
ENDOPROTHÈSE EXPANSIBLE

(30) Priorität: 10.09.2015 DE 102015115279
(43) Veröffentlichungstag der Anmeldung: 18.07.2018
(73) Patentinhaber: Bentley InnoMed GmbH, 72379 Hechingen (DE)
(72) Erfinder: OBRADOVIC, Milisav, 79539 Lörrach (DE)
(74) Vertreter: Hoefer & Partner Patentanwälte mbB
(86) Internationale Anmeldenummer: PCT/EP2016/071296
(87) Internationale Veröffentlichungsnummer: WO 2017/042329

(56) Entgegenhaltungen:
- WO-A1-03/047463
- US-A1- 2001 044 652
- US-A1- 2004 133 271
- US-A1- 2007 168 010
- US-A1- 2014 025 161

## Beschreibung

Die Erfindung betrifft eine Gefäßstütze mit einzelnen Ringsegmenten, deren Stege einen mäandrierenden Verlauf nehmen, und Verbindungsstegen zwischen benachbarten Ringsegmenten, die in Verbindungspunkten mit den Stegen der Ringsegmente zusammenlaufen. Die Gefäßstützen bestehen insbesondere aus einem in vivo abbaubaren Material und ganz besonders bevorzugt aus einem solchen Kunststoff.

Gefäßstützen werden vielfach im koronaren, zerebralen und peripheren Bereich zur Erweiterung und Rekanalisierung von verengten Gefäßen und zur Abschottung von Aneurysmen, Shunts und unerwünschten Abzweigungen in Gefäßen eingesetzt. Dabei wird zunehmend die Verwendung in vivo abbaubarer Materialien gesetzt, die sich über einen definierten Zeitraum im Körper auflösen. Gefäßstützen, insbesondere auch medikamentös aufgeladene Stents, werden zumeist nur über einen begrenzten Zeitraum benötigt und sind entbehrlich, wenn sie ihren Zweck erfüllt haben.

Für in vivo abbaubaren Gefäßstützen wurden eine große Anzahl an Materialien getestet, sowohl metallische als auch solche auf Kunststoffbasis. Bei den Metallen haben sich insbesondere Magnesiumlegierungen als brauchbar erwiesen. Unter den Kunststoffen wurden u.a. Gefäßstützen auf Basis von Polylactiden und Polygalactiden entwickelt.

Gefäßstützen, die der Erweiterung von Gefäßen dienen, werden zumeist auf Ballone aufgekrimpt über Katheter platziert, mithilfe der Ballone expandiert, wonach die Ballone in relativiertem Zustand unter Zurücklassung der Gefäßstütze zurückgezogen werden. Bei der Expansion der Gefäßstützen werden einzelnen Ringsegmente mehr oder weniger stark aufgeweitet und geraten dabei unter Spannung. Gleichzeitig führt die Aufweitung aber dazu, dass die unter Spannung stehenden Teile des Stents sich aus der Zylinderebene heraus bewegen. Dabei entstehende Kanten und Grate können zu Gefäßverletzungen führen.

Dieser Gefahr kann durch die Verwendung von Kunststoffen zumindest teilweise begegnet werden. Kunststoffe sind weniger hart als Metalle und neigen weniger zur Ausbildung von scharfen Kanten. Ein Nachteil von Kunststoffstents ist aber ihre Tendenz, bei der Aufweitung an den auf Zug beanspruchten Stellen Risse auszubilden bzw. im Falle einer Stauchung sich stark aufzuwölben. Gerade bei Gefäßstützen, sich auf einer Mehrzahl von Ringsegmenten mit mäandrierenden Stegen zusammensetzen, ist dieses Verhalten häufig. Andererseits haben sich Stentstrukturen dieser Art aufgrund ihres Radialverhaltens und der Beherrschbarkeit der Längenreduktion bei der Expansion als erfolgreich erwiesen.

Weiterhin gibt es verschiedene Ansätze, das Expansionsverhalten von Stents konstruktiv zu verbessern. So zeigen beispielsweise die US2004/0133271A1 und die US2001/044652A1 jeweils Stentdesigns mit Einschnitten oder Kerben in den Stegen. Es bleibt jedoch weiterhin der Bedarf nach neuen Designs mit optimiertem Expansionsverhalten.

Angesichts dessen ist es Aufgabe der Erfindung, Strukturen bereit zu stellen, mit denen sich die oben beschriebenen Spannungen, wie sie bei der Expansion einer Gefäßstütze auftreten, zu reduzieren. Dies gilt nicht nur für die besonders betroffenen Kunststoffstents, sondern auch für Stents aus metallischen Materialien, seien sie in vivo abbaubaren oder nicht.

Diese Aufgabe wird mit einer Gefäßstütze gemäß Anspruch 1 gelöst.

Erfindungsgemäß werden die Begriffe "Stent" und "Gefäßstütze" synonym verwandt. Die Begriffe erstrecken sich nicht nur auf vasculäre und coronare Stents, sondern vielmehr auf alle in Körperlumina einsetzbare Gefäßstützen.

Der Begriff "Ringsegment" bezeichnet in Umfangsrichtung verlaufende Stützelemente einer Gefäßstütze. Solche Ringsegmente haben einen mäandrierenden Verlauf, damit bei der Expansion durch Streckung der Schlingen der Durchmesser der Gefäßstütze erweitert werden kann. "Mäandrierenden" bezeichnet dabei einen Verlauf mit Krümmungen, in Schlangenlinien oder in Zickzackform.

Die einzelnen Ringsegmente einer Gefäßstütze bestehen jeweils, je nach Betrachtungsweise, aus einem einzigen Steg mit gekrümmtem Verlauf oder einer Vielzahl von Stegen, die jeweils miteinander verbunden sind und die mäandrierenden Form ausbilden.

Aneinandergrenzende Ringsegmente einer Gefäßstütze sind durch Verbindungsstege miteinander verbunden. Die Verbindungstege können einen geraden, gekrümmten, S-förmigen oder auch spiralförmigen Verlauf haben; Verbindungstege mit geradem Verlauf sind erfindungsgemäß bevorzugt.

In der Regel ist es erwünscht, die Längenkontraktion eines Stents bei der Expansion zu begrenzen. Dies wird bei Verwendung gekrümmter Verbindungsstege durch Streckung der Stege erreicht, bei Verwendung gerader Verbindungsstege durch deren Anordnung zwischen den Außenbögen eines

Ringsegments und den Innenbögen des benachbarten Ringsegments. Die Verbindungstege sind damit zwischen den jeweiligen Wendepunkten benachbarter Mäandern angeordnet, ein Ball auf der Außenseite, zum anderen auf der Innenseite.

Die Stege der Ringsegmente einerseits und die Verbindungsstege andererseits treffen sich auf beiden Seiten der Verbindungsstege in Verbindungspunkten. In der oben beschriebenen Konstellation gehen von den Verbindungspunkten jeweils drei Stegarme aus.

Erfindungsgemäß befinden sich an einigen der Verbindungspunkte von außen in den Steg hineinreichende Aussparungen, bevorzugt an den Verbindungspunkten, an denen im Bereich der Einmündungen der Verbindungsstege die Ringsegmente gestaucht werden. Dies ist in der Regel bei den Verbindungspunkten an den Außenbögen der Fall. Die Aussparungen geben dem gestauchten Material des Ringsegments Platz zum Ausweichen, sodass es nicht zu den oben beschriebenen Aufwölbungen kommt.

Bei den Aussparungen handelt es sich um Kerben oder Einschnitte, die von außen in den Steg hinein führen, aber auch um regelrechte Ausnehmungen oder "Ausstanzungen". Die Aussparungen haben eine gerundete Kontur, was den Vorteil mit sich bringt, dass Rissansätze, wie sie beim Aufkrimpen auf einen Ballon und Expandieren des Ballons auftreten können, stillgelegt werden. Besonders bevorzugt sind Ausnehmungen mit kreisförmiger oder elliptischer Kontur. Die Aussparungen reichen durch den Steg hindurch und sind zum Rand hin offen.

Die Aussparungen erstrecken sich im Bereich der Winkel der Verbindungspunkte von außen in die Ringstege hinein. Sie können dabei auch die Verbindungsstege erfassen. Bei kreisförmigen oder elliptischen Aussparungen beträgt der Radius vorzugsweise 1/20 bis 1/10 der Stegbreite. Für Einschnitte und Kerben gilt entsprechendes.

Alternativ oder zusätzlich können die Stege der Ringsegmente Einschnitte aufweisen, die sich im Bereich der Bögen der mäandrierenden Ringsegmente befinden. Bei den Einschnitten handelt es sich um Schlitze, die dem Verlauf des Ringsegments folgen. Einschnitte bzw. Schlitze befinden sich vollständig im Innern der Stege. Ihre Breite beträgt vorzugsweise 1/40 bis 1/4, insbesondere 1/20 bis 1/10 der Stegbreite. Die Einschnitte geben den Ringsegmenten Flexibilität und sind geeignet, das bei einer Stauchung verdrängte Material aufzunehmen.

Die Einschnitte oder Schlitze befinden sich vorzugsweise im Bereich der Verbindungspunkte der Ringsegmente, an denen Verbindungsstege in Innenbögen angeordnet sind. Hier weisen die Ringsegmente auf der den Verbindungsstegen gegenüberliegenden Seite eine Einbuchtung oder Mulde auf, deren Kontur die Einschnitte folgen.

In der Regel erstrecken sich die Einschnitte über eine Länge, die der einfachen bis dreifachen Stegbreite entspricht.

Einschnitte oder Schlitze können insbesondere auch im Bereich der Bögen der Ringsegmente angeordnet sein, wo sie dem Stegverlauf folgen.

Die erfindungsgemäße Gestaltung der Gefäßstützen ist besonders für solche geeignet, die aus einem Kunststoff geschnitten sind, etwa aus einem abbaubaren Kunststoff. Als solche kommen insbesondere Polylactide, Polygalactide, deren Copolymere und deren Mischungen infrage. Es versteht sich aber, dass die Erfindung Gefäßstützen aus beliebigen Materialien anwendbar ist; auf das Material kommt es nicht an.

Bei den erfindungsgemäßen Stents handelt es sich um übliche ballonexpandierbare Stents. Diese werden auf übliche Weise auf einen Ballon gekrimpt, über einen Ballonkatheter platziert, hydraulisch expandiert, wonach der Katheter mit dem entspannten Ballon zurückgezogen wird. Es versteht sich, dass der Stent auf an und für sich bekannte Weise mit Wirkstoffen beschichtet oder getränkt sein kann, beispielsweise um bei verengten Gefäßen eine Restenose zu verhindern.

Die Erfindung wird durch die beiliegenden Abbildungen näher erläutert. Es zeigen:
- Fig. 1: ein herkömmliches Stentdesign in ausgebreiteter Form;
- Fig. 2: einen Ausschnitt aus dem Stentdesign von Fig. 1, erfindungsgemäß modifiziert;
- Fig. 3: Modifikationen des Designs von Fig. 2 bezüglich der Aussparungen und Einschnitte;
- Fig. 4: weitere Modifikationen des Designs von Fig. 2;
- Fig. 5: weitere Modifikationen des Designs von Fig. 2 mit Einschnitten;
- Fig. 6: eine fotografische Darstellung eines Musters gemäß Fig. 2 vor der Expansion des Stents; und
- Fig. 7: den Ausschnitt von Fig. 6 nach der Expansion des Stents.

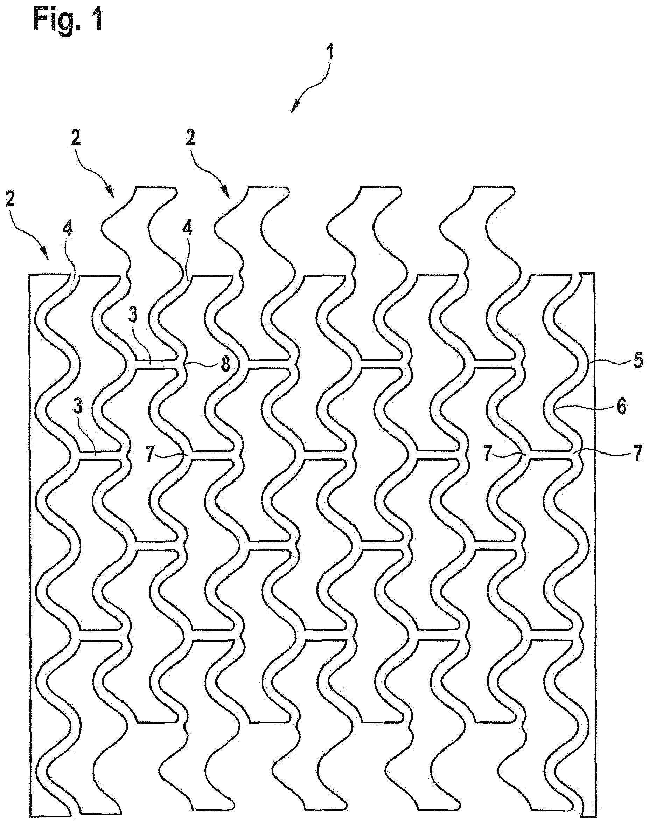

Figur 1 zeigt einen herkömmlichen Stent 1 mit mäandrierenden Ringsegmenten 2 und Verbindungsstegen 3 zwischen benachbarten Ringsegmenten 2 in ausgeschnittener und ausgebreiteter Form. Die einzelnen Ringsegmente bestehen aus einem durchgehenden Steg 4, der einen mäandrierenden oder schlangenlinienförmigen Verlauf nimmt, mit Außenbögen 5, Innenbögen 6 und Verbindungspunkten 7 zu Verbindungsstegen 3. Die Innenbögen 6 an den Verbindungspunkten 7 zu den Verbindungsstegen 3 weisen eine muldenförmige Eintiefung 8 auf. Die Verbindungsstege 3 haben eine Breite, die geringfügig geringer ist, als die Stege der Ringsegmente 2, etwa um 5 bis 20 %.

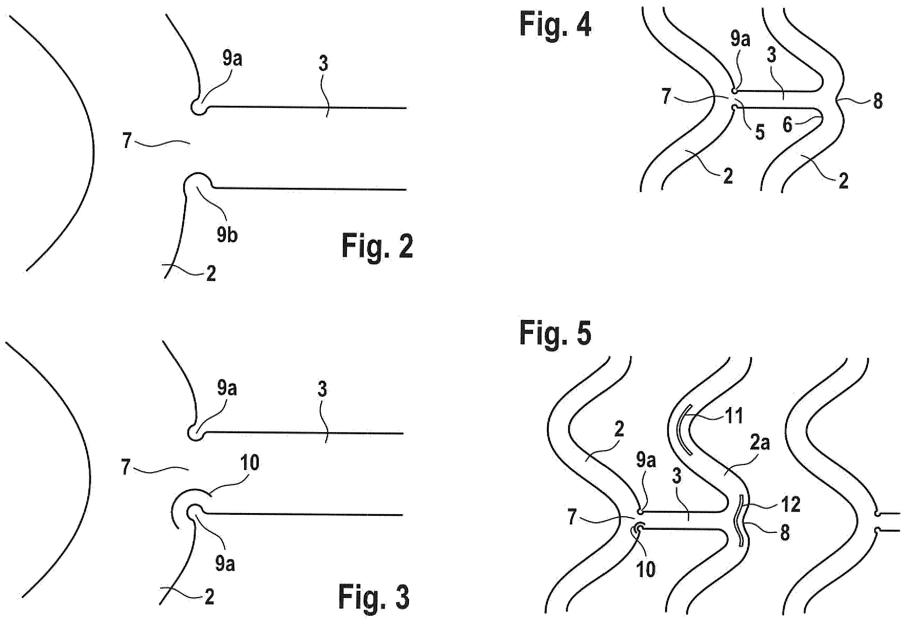

Figur 2 zeigt einen Ausschnitt aus dem Stentdesign von Figur 1 mit erfindungsgemäßen Aussparungen 9a und 9b. Die Aussparungen 9a und 9b stellen zwei unabhängige Varianten dar, die jede für sich verwirklicht werden können und in den Winkeln des Verbindungpunkts 7 zwischen dem Ringsegment 2 und dem Verbindungsglied 3 angeordnet sind. Beide Aussparungen 9a und 9b haben eine kreisförmige Kontur und weisen einen gerundeten Übergang in die Ränder des Ringsegments 2 und des Verbindungsstegs 3 auf. Beide Aussparungen 9a und 9b sind so angeordnet, dass sie sowohl den Verbindungssteg 3 als auch das Ringsegment 2 erfassen. Aussparung 9b hat einen größeren Durchmesser als Aussparung 9a.

Figur 3 zeigt die erfindungsgemäße Gestaltung der Verbindungspunkte 7 mit Aussparungen 9a in den Winkeln, die zwischen den Verbindungsstegen 3 und dem Ringsegmente 2 gebildet sind. Um eine der Aussparungen 9a herum ist ein Einschnitt 10 angeordnet, der die Aussparung 9a nahezu halbkreisförmig umgibt.

Die Aussparungen 9a und 9b der Figuren 1 und 2 wie auch der Einschnitt 10 befinden sich in den Bereichen des Stents 1, die bei der Expansion unter Druckspannung geraten. Die Aussparungen stellen Freiraum dar, in den bei der

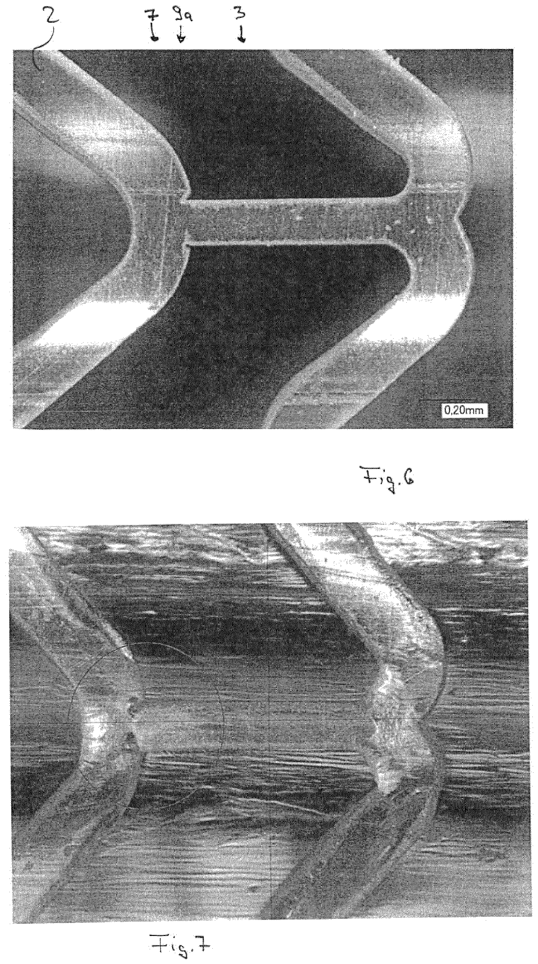

Expansion Material verlagert werden kann, siehe Figur 6/7. Gleichzeitig sind die gerundeten Konturen geeignet, einer Rissbildung vorzubeugen.

Figur 4 zeigt eine weitere Gestaltung eines erfindungsgemäßen Stents mit im Verbindungspunkt. 7 angeordneten Aussparungen 9a. Die halbkreisförmigen Einschnitte 10 sind in der Abbildung nicht gezeigt. Der Verbindungssteg 3 erstreckt sich vom Außenbogen 5, der zugleich Verbindung Punkt 7 ist, zum Innenbogen 6 des benachbarten Ringsegments 2. Gegenüber dem Steg 3 und dem Innenbogen 6 ist das Ringsegment 2 mit einer Einwölbung oder Mulde 8 ausgestattet. Eine solche Muldenbildung verlängert den Innenbogen 6 und erlaubt es, den Übergang vom Verbindungssteg 3 zum Ringsegment 2 sanfter zu runden.

Figur 5 zeigt eine weitere Variante einer erfindungsgemäßen Gestaltung eines Stents 1 im Bereich eines Verbindungsstegen 3 zwischen 2 Ringsegmenten 2. Aussparungen 9a im Bereich des Verbindungpunkts sind halbkreisförmig mit einem Schlitz 10 umgeben sein. Gleichzeitig befinden sich Schlitz förmige Einschnitte 11 und 12, die dem Verlauf des Ringsegments 2 Folgen, auf dem Ringsegmente 2a, dass du dem Ringsegments 2 mit den Aussparungen 9a benachbart ist. Die Schlitze sind geeignet, die bei der Expansion des Stents auftretenden Spannungen auszugleichen.

Figur 6 zeigt eine fotografische Aufnahme eines Stents aus Schnitz mit Aussparungen 9 klein A Bereich des Verbindungpunkts 7 zwischen Ringsegments 2 und Steg 3. Die halbkreisförmigen Einschnitte 10 sind nicht gezeigt. Figur 7 zeigt den gleichen Bereich des Stents von Figur 6 nach der Expansion des Stents; die Aussparungen haben sich durch Verlagerung von Material aus dem Ringsegment 2 geschlossen.

Es versteht sich, dass die in den Abbildungen gezeigten und in der Beschreibung dazu erläuterten Merkmale jeweils für sich stehen und auch in anderem Zusammenhang eingesetzt werden können. So sind in einem Stent beliebige Kombinationen von Einschnitten und/oder Aussparungen möglich.

## Patentansprüche

1. Gefäßstütze, insbesondere aus einem in vivo abbaubaren Kunststoff mit einzelnen Ringsegmenten (2), deren Stege (4) einen mäandrierenden Verlauf nehmen, und Verbindungsstegen (3) zwischen benachbarten Ringsegmenten (2), die in Verbindungspunkten (7) mit den Stegen (4) der Ringsegmente (2) zusammenlaufen, wobei in Winkeln von Verbindungspunkten (7), die bei der Expansion der Gefäßstütze (1) gestaucht werden, von außen in die Ring- (4) und Verbindungsstege (3) hinein- und durch die Ring- (4) und Verbindungsstege (3) hindurchreichende Aussparungen (9a, 9b) angeordnet sind, die zum Rand hin offen sind und gerundete Konturen aufweisen, um bei der Expansion der Gefäßstütze (1) auftretende Spannungen zu reduzieren, **dadurch gekennzeichnet, dass** in Winkeln von Verbindungspunkten (7), in denen Aussparungen (9a, 9b) angeordnet sind, um die Aussparungen (9a, 9b) herum durch die Stege (4, 3) hindurchreichende Einschnitte (10) angeordnet sind, die die Aussparungen (9a, 9b) nahezu halbkreisförmig umgeben.

2. Gefäßstütze nach Anspruch 1, **dadurch gekennzeichnet, dass** die Aussparungen (9a, 9b) im Wesentlichen kreisförmig oder elliptisch ausgebildet sind.

3. Gefäßstütze nach Anspruch 1 oder 2, **dadurch gekennzeichnet, dass** die Aussparungen (9a, 9b) einen Radius von 1/20 bis 1/10 der Stegbreite der Ringsegmente haben.

4. Gefäßstütze nach einem oder mehreren der vorstehenden Ansprüche, **dadurch gekennzeichnet, dass** die Stege (4) der Ringsegmente (2) Einschnitte (11, 12) aufweisen, die dem Verlauf der Stege folgen.

5. Gefäßstütze nach Anspruch 4, **dadurch gekennzeichnet, dass** die Einschnitte (11, 12) in den Bereichen der Stege angeordnet sind, die bei der Expansion der Gefäßstütze gestaucht werden.

6. Gefäßstütze nach Anspruch 4 oder 5, **dadurch gekennzeichnet, dass** die Einschnitte (10, 12) im Bereich der Verbindungspunkte (7) angeordnet sind.

7. Gefäßstütze nach Anspruch 4 oder 5, **dadurch gekennzeichnet, dass** die Einschnitte (11) im Bereich der Bögen der Ringsegmente angeordnet sind.

8. Gefäßstütze nach einem oder mehreren der vorhergehenden Ansprüche, **dadurch gekennzeichnet, dass** die Einschnitte (10, 11, 12) Schlitze mit einer Breite von 1/40 bis 1/4, insbesondere 1/20 bis 1/10 der Stegbreite sind.

9. Gefäßstütze nach einem oder mehreren der vorstehenden Ansprüche, **dadurch gekennzeichnet, dass** die Verbindungsstege (3) Außenbögen von Ringsegmenten (2) mit Innenbögen benachbarter Ringsegmente (2) verbinden.

10. Gefäßstütze nach Anspruch 9, **dadurch gekennzeichnet, dass** die Bögen der Ringsegmente (2), in deren Innenbögen Verbindungsstege (3) einmünden, gegenüber der Einmündung eine muldenförmige Eintiefung (8) aufweisen.

11. Gefäßstütze nach Anspruch 10, **dadurch gekennzeichnet, dass** die Einschnitte (12) dem Verlauf der muldenförmige Eintiefung (8) folgen.

12. Gefäßstütze nach einem oder mehreren der vorstehenden Ansprüche, **dadurch gekennzeichnet, dass** sie aus einem in vivo abbaubaren Kunststoff besteht und mit einem Ballon expandierbar ist.

13. Gefäßstütze nach Anspruch 12, **dadurch gekennzeichnet, dass** die Gefäßstütze aus Polylactid, Polygalactid oder Mischungen oder Copolymeren derselben besteht.

## Claims

1. Vascular stent, in particular made of an in vivo degradable plastic material, having individual ring segments (2), the webs (4) thereof are of meandering configuration, and comprising connecting webs (3) arranged between adjacent ring segments (2), said webs converging in connection points (7) with the webs (4) of the ring segments (2), wherein
recesses (9a, 9b) are arranged at angles of the connection points (7) being compressed when the vascular stent (1) is expanded, said recesses having rounded contours and being open towards the edge and extending from the edge into the ring segment webs (4) and connecting webs (3) and through the ring segment webs (4) and connecting webs (3), in order to reduce stresses arising during the expansion of the vascular stent (1),
**characterized in that** at angles of the connection points (7) where recesses (9a, 9b) are arranged, incisions (10) extending through the webs (4, 3) are arranged close to semicircularly around the recesses (9a, 9b).

2. Vascular stent according to claim 1, **characterized in that** the recesses (9a, 9b) are substantially of circular or elliptical shape.

3. Vascular stent according to claim 1 or 2, **characterized in that** the recesses (9a, 9b) have a radius of 1/20 to 1/10 of the width of the ring segments (2).

4. Vascular stent according to any one of the preceding claims, **characterized in that** the webs (4) of the ring segments (2) are provided with incisions (11, 12) which follow the course of the webs.

5. Vascular stent according to claim 4, **characterized in that** the incisions (11, 12) are arranged in the regions of the webs that are compressed during the expansion of the vascular stent.

6. Vascular stent according to claim 4 or 5, **characterized in that** the incisions (10, 12) are arranged in the region of the connecting points (7).

7. Vascular stent according to claim 4 or 5, **characterized in that** the incisions (11) are arranged in the region of the arches of the ring segments.

8. Vascular stent according to any one of the preceding claims, **characterized in that** the incisions (10, 11, 12) are slots having a width of 1/40 to 1/4 in particular 1/20 to 1/10 of the web width.

9. Vascular stent according to any one of the preceding claims, **characterized in that** the connecting webs (3) connect outer arches of ring segments (2) with inner arches of adjacent ring segments (2).

10. Vascular stent according to claim 9, **characterized in that** the arches of the ring segments (2) into whose inner arches the connecting webs (3) adjoin are provided with a trough-like depression (8) at a location opposite to the junction point.

11. Vascular stent according to claim 10, **characterized in that** the incisions (12) follow the course of the trough-like depression (8).

12. Vascular stent according to any one of the preceding claims **characterized in that** said stent consists of an in vivo degradable plastic material and is expandable by means of a balloon.

13. Vascular stent according to claim 12, **characterized in that** the vascular stent consists of polylactide, polygalactide or blends or copolymers thereof.

## Revendications

1. Endoprothèse, en particulier composée d'une matière plastique dégradable in vivo avec des segments annulaires (2) individuels, dont les entretoises (4) adoptent un profil sous forme de méandre, et des entretoises de liaison (3) entre des segments annulaires (2) adjacents, qui convergent en des points de liaison (7) avec les entretoises (4) des segments annulaires (2), dans laquelle sont disposés, à des angles par rapport à des points de liaison (7), qui sont aplatis lors de l'expansion de l'endoprothèse (1), des évidements (9a, 9b) parvenant à l'intérieur des entretoises annulaires (4) et des entretoises de liaison (3) depuis l'extérieur et traversant les entretoises annulaires (4) et les entretoises de liaison (3) de part en part, lesquels sont ouverts en direction du bord et présentent des contours arrondis pour réduire des tensions apparaissant lors de l'expansion de l'endoprothèse (1), **caractérisée en ce que** sont disposées, à des angles par rapport à des points de liaison (7), dans lesquels des évidements (9a, 9b) sont disposés, des incisions (10) traversant les entretoises (4, 3) de part en part tout autour des évidements (9a, 9b), qui entourent quasiment en forme de demi-cercle les évidements (9a, 9b).

2. Endoprothèse selon la revendication 1, **caractérisée en ce que** les évidements (9a, 9b) sont réalisés sensiblement en forme de cercle ou de manière elliptique.

3. Endoprothèse selon la revendication 1 ou 2, **caractérisée en ce que** les évidements (9a, 9b) présentent un rayon de 1/20 à 1/10 de la largeur d'entretoise des segments annulaires.

4. Endoprothèse selon l'une quelconque ou plusieurs des revendications précédentes, **caractérisée en ce que** les entretoises (4) des segments annulaires (2) présentent des incisions (11, 12), qui suivent le profil des entretoises.

5. Endoprothèse selon la revendication 4, **caractérisée en ce que** les incisions (11, 12) sont disposées dans les zones des entretoises, qui sont aplaties lors de l'expansion de l'endoprothèse.

6. Endoprothèse selon la revendication 4 ou 5, **caractérisée en ce que** les incisions (10, 12) sont disposées dans la zone des points de liaison (7).

7. Endoprothèse selon la revendication 4 ou 5, **caractérisée en ce que** les incisions (11) sont disposées dans la zone des arcs des segments annulaires.

8. Endoprothèse selon l'une quelconque ou plusieurs des revendications précédentes, **caractérisée en ce que** les incisions (10, 11, 12) sont des entailles avec une largeur de 1/40 à 1/4, en particulier de 1/20 à 1/10 de la largeur d'entretoise.

9. Endoprothèse selon l'une quelconque ou plusieurs des revendications précédentes, **caractérisée en ce que** les entretoises de liaison (3) relient des arcs extérieurs de segments annulaires (2) à des arcs intérieurs de segments annulaires (2) adjacents.

10. Endoprothèse selon la revendication 9, **caractérisée en ce que** les arcs des segments annulaires (2), dans les arcs intérieurs desquels débouchent des entretoises de liaison (3), présentent par rapport à l'embouchure un renfoncement (8) en forme de cavité.

11. Endoprothèse selon la revendication 10, **caractérisée en ce que** les incisions (12) suivent le profil du renfoncement (8) en forme de cavité.

12. Endoprothèse selon l'une quelconque ou plusieurs des revendications précédentes, **caractérisée en ce qu'**elle est constituée d'une matière plastique dégradable in vivo et peut s'expanser avec un ballonnet.

13. Endoprothèse selon la revendication 12, **caractérisée en ce que** l'endoprothèse est constituée de polylactide, de polygalactide ou de mélanges ou de copolymères de ceux-ci.
